# EUROPEAN PATENT SPECIFICATION

(11) **EP 3 291 811 B1**
(45) Date of publication and mention of the grant of the patent: **07.08.2019**
(21) Application number: 16719430.7
(22) Date of filing: 02.05.2016
(51) Int. Cl.: A61K 31/426, A61K 31/427, A61K 31/4439, A61K 31/506, A61K 31/519, A61K 31/5377, A61K 31/635, A61K 45/06, A61P 17/02, A61K 31/197, A61K 31/53

(54) **THE USE OF SGC STIMULATORS, SGC ACTIVATORS, ALONE AND COMBINATIONS WITH PDE5 INHIBITORS FOR THE TREATMENT OF DIGITAL ULCERS (DU) CONCOMITANT TO SYSTEMIC SCLEROSIS (SSC)**
VERWENDUNG VON SGC-STIMULATOREN, SGC-AKTIVATOREN, ALLEINE UND IN KOMBINATION MIT PDE5-HEMMERN ZUR BEHANDLUNG VON DIGITALEN GESCHWÜREN MIT SYSTEMISCHER SKLEROSE (SSC)
UTILISATION DE STIMULATEURS DE LA SGC, D'ACTIVATEURS DE LA SGC, SEULS ET EN COMBINAISONS AVEC DES INHIBITEURS DE PDE5 POUR LE TRAITEMENT D'ULCÈRES DIGITAUX (DU) ASSOCIÉS À LA SCLÉRODERMIE SYSTÉMIQUE (SSC)

(30) Priority: 06.05.2015 EP 15166516
(43) Date of publication of application: 14.03.2018
(73) Proprietor: Bayer Pharma Aktiengesellschaft, 13353 Berlin (DE)
(72) Inventor: HIRTH-DIETRICH, Claudia, 42115 Wuppertal (DE); SANDNER, Peter, 42113 Wuppertal (DE); STASCH, Johannes-Peter, I-00046 Grottaferrata (IT); HAHN, Michael, 40764 Langenfeld (DE); FOLLMANN, Markus, 50859 Köln (DE); VAKALOPOULOS, Alexandros, 40721 Hilden (DE)
(74) Representative: BIP Patents
(86) International application number: PCT/EP2016/059734
(87) International publication number: WO 2016/177660

(56) References cited:
- WO-A1-2011/147810
- US-A1- 2009 215 769
- US-A1- 2009 221 573
- Anonymous: "Bayer to Evaluate sGC Stimulator Riociguat in Patients With Diffuse Cutaneous Systemic Sclerosis - News Release", Bayer , 13 November 2014 (2014-11-13), XP002759127, Retrieved from the Internet: URL:http://www.epresspack.net/bayer-riocig uat/bayer-to-evaluate-sgc-stimulator-rioci guat-in-patients-with-diffuse-cutaneous-sy stemic-sclerosis/ [retrieved on 2016-06-22]
- Anonymous: "Digital ulcers: sildenafil", Nice , 24 March 2015 (2015-03-24), XP002759128, Retrieved from the Internet: URL:https://www.nice.org.uk/guidance/esuom 42/resources/digital-ulcers-sildenafil-541 16459119149253 [retrieved on 2016-06-22]
- S. KOTZKI ET AL: "Anodal Iontophoresis of a Soluble Guanylate Cyclase Stimulator Induces a Sustained Increase in Skin Blood Flow in Rats", JOURNAL OF PHARMACOLOGY AND EXPERIMENTAL THERAPEUTICS, vol. 346, no. 3, 9 July 2013 (2013-07-09), pages 424-431, XP055282639, DOI: 10.1124/jpet.113.205484
- DEES CLARA ET AL: "Stimulators of soluble guanylate cyclase (sGC) inhibit experimental skin fibrosis of different aetiologies", ANNALS OF THE RHEUMATIC DISEASES, BRITISH MEDICAL ASSOCIATION, LONDON, GB, vol. 74, no. 8, 1 January 2015 (2015-01-01), pages 1621-1625, XP008180685, ISSN: 0003-4967
- BEYER CHRISTIAN ET AL: "Stimulation of soluble guanylate cyclase reduces experimental dermal fibrosis", ANNALS OF THE RHEUMATIC DISEASES, BRITISH MEDICAL ASSOCIATION, LONDON, GB, vol. 71, no. 6, 1 June 2012 (2012-06-01), pages 1019-1026, XP009167097, ISSN: 0003-4967
- SANDNER PETER ET AL: "Stimulators of Soluble Guanylate Cyclase (sGC) Improve Wound Healing in the Tsk-1 Mouse Skin Fibrosis Mode", ARTHRITIS & RHEUMATOLOGY, vol. 67, no. Suppl. 10, ABS.1906, October 2015 (2015-10), XP002759129, & ANNUAL MEETING OF THE AMERICAN-COLLEGE-OF-RHEUMATOLOGY (ACR) AND ASSOCIATION-OF-RHEUMATOLOGY-HEALTH-; SAN FRANCISCO, CA, USA; NOVEMBER 06 -11, 2015
- M. Ollé: "The European League Against Rheumatism (EULAR) - 16th Annual European Congress (June 10-13, 2015 - Rome, Italy).", Drugs of Today, vol. 51, no. 6 June 2015 (2015-06), June 2015 (2015-06), pages 387-392, XP002759130, Retrieved from the Internet: URL:http://www.ncbi.nlm.nih.gov/pubmed/262 61853 [retrieved on 2016-06-22]

## Description

The use of sGC stimulators, sGC activators alone, or in combination with PDE5 inhibitors for the prevention and healing of Digital Ulcers which are concomitant to fibrotic diseases, such as systemic sclerosis and scleroderma.

### Background of the invention

### Systemic Sclerosis and concomitant Digital Ulcers (DU)

The pathogenesis of Systemic Sclerosis (SSc) is still unclear and remains elusive. However, scleroderma is a non-inherited, noninfectious disease and thought to be an autoimmune disease. SSc has a broad variety of symptoms triggered by excessive deposition of extracellular matrix in the dermis resulting in skin fibrosis. In later stages SSc is characterized by progressive tissue fibrosis affecting other internal organs as the gut, the lung or the kidneys. Therefore scleroderma is the hallmark of the disease comprising also e.g. lung fibrosis, renal fibrosis, fibrosis of the heart, the gut or the blood vessels. Besides excessive fibrosis in the skin and internal organs, SSc is also characterized by vasculopathies and microangiopahties. Especially small vessel vasculopathies and concomitant vascular malperfusion and ischemia can cause Raynaud's phenomena (RP) but also to the formation of digital ulcer (DU). Whereas tissue fibrosis can cause end organ failure and lead to high morbidity and mortality in patients with end-stage SSc, formation of DU substantially reduce the quality of life of SSc patients, impairs hand function and leads to disability. (Harris et al. 2005 - Kelley's Textbook of Rhematology 7th edition. Elsevier Saunders, Philadelphia PA).

There is still no causative treatment for Systemic Sclerosis (SSc) available and the current therapy is based on suppression of the immune system via corticosteroids, cyclophosphamide, methotrexate. More recently kinase inhibitors and anti-inflammatory drugs are under investigation as immunosuppressant and antifibrotic agents in SSc, but tolerability is limited in SSc patients (Khanna and Denton 2010 - Best. Pract. Res. Clin. Rheumatol. 24:387-400, Ong and Denton 2010 - Curr. Opin. Rheumatol. 22:264-272, Spiera 2011 - Ann. Rheum. Dis. Epub Mar 2011). These therapies either used as stand alone treatment or combined are of limited efficacy and exhibited considerable side effects. Therefore alternative treatment options in SSc which are efficacious and safe are urgently needed. In addition, there is currently no approved treatment for healing of DU but vasoactive drugs as prostacyclin agonists and endothelin antagonists are used.

### Antifibrotic effects of cGMP:

The cyclic nucleotides, cyclic adenosine monophosphate (cAMP) and cyclic guanosine monophosphate (cGMP), were discovered decades ago and represent one of the most important second messenger pathway within cells. It is well established that the regulation of intra-cellular cGMP pools have substantial impact on physiology, and pathophysiology and is one basic principle of pharmacological intervention (Evgenov et al. 2006 - Nat. Rev. Drug. Discov. 5(9):755-768). Besides the treatment of cardiovascular, lung or CNS-disorders there is ample evidence that an increase in cGMP is a very effective treatment option for urological disorders as well (Sandner et al. 2009 - Handbook Exper. Pharmacol. 191:507-531). PDE5 inhibitors are the gold-standard for the treatment of erectile dysfunction (ED) but it was shown that PDE5 inhibitors could be useful for the treatment of symptomatic BPH which is characterized by Overactive Bladder (OAB) and Lower Urinary Tract Symptoms (LUTS) (Porst et al. 2008 - Curr. Urol. Rep. 9:295-301; .McVary et al. 2007 - J. Urol. 177:1071-1077, J Urol. 177:1401-1407, Kaplan and Gonzalez. 2007 - Rev. Urol. 9:73-77). The antifibrotic effects of Vardenafil, sGC stimulators and sGC activators is not understood yet. There are some descriptions about antifibrotic effects of Nitric-Oxide which are presumably mediated by cGMP in other organs and PDE5 inhibitors or guanylate cyclase stimulators have shown efficacy in penile fibrosis (Peyronie's disease) (Ferrini et al. 2006 - B. J. Urol. 97:625-633) and liver fibrosis (Knorr et al. 2008 - Arzneimittelforschung 58:71-80) respectively.

The document News Release: "Bayer to Evaluate sGC Stimulator Riociguat in Patients With Diffuse Cutaneous Systemic Sclerosis",, 13 November 2014 (2014-11-13), (http://www.epresspack.net/bayer-riociguat/bayer-to-evaluate-sgc-stimulator-riociguat-in-patients-with-diffuse-cutaneous-systemic-sclerosis/) reports the initiation of a Phase II study to investigate the efficacy and safety of Riociguat in the treatment of diffuse cutaneous systemic sclerosis.

It was not known if the NO/cGMP system is involved in SSc and if cGMP increase provides a treatment option for this disease. We hypothetized that - independent from endogenous NO/cGMP production - sGC stimulators and activators might be an effective treatment option for Systemic Sclerosis (SSc) by reduction of skin fibrosis. In WO2011/147810 we have recently shown that sGC stimulators, sGC activators, alone and combinations with PDE5 inhibitors could directly target skin fibrosis which is one hallmark of Systemic Sclerosis (SSc). This clearly demonstrated that sGC stimulators, sGC activators, alone and combinations with PDE5 inhibitors are an effective future treatment option for SSc. However, it is not known if the vasculopathies in SSc patients which lead e.g. to DU formation which are one of the most bothersome symptoms in SSc, could be also efficiently treated with sGC stimulators, sGC activators, alone and combinations with PDE5 inhibitors. Since these compounds can induce peripheral vasodilation it could be assumed that SSc driven vasculopathies might be reduced, preventing new formation of DU. However, it was unclear if SSc-driven DU could be also healed giving the antifibrotic mode of action of sGC stimulators/sGC activators alone and in combination with PDE5 inhibitors. Therefore, increased blood flow may be counteracted by reduced collagen-synthesis or synthesis of extracellular matrix which is necessary for wound closure and which then may impair wound healing in SSc patients.

We therefore investigated sGC stimulators and sGC activators, i.e. compound of the formula 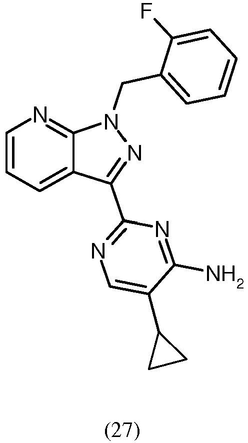 and combinations with PDE5 inhibitors thereof on wound healing was in TSK mice an animal model for SSc characterized by excessive skin fibrosis We found in vivo in our animal models that:
- TSK mice have an attenuated wound healing compared to WT mice.
- sGC stimulators or sGC activators, i.e. compounds according to formulae (27) and (3) significantly and dose-dependently accelerated wound healing in the TSK mice.
- sGC stimulators or sGC activators, i.e compounds according to formulae (27) and (3) normalized the healing time to healthy WT control mice. These data suggest that despite the antifibrotic effectof sGC stimulators and sGC activators in SSc, wound healing in SSc could be significantly accelerated and normalized to the levels of healthy control individuals

In summary, we found completely unexpected and for the first time that sGC stimulators or sGC activators i.e. compounds according to formulae (27) and (3), which prevent fibrosis and regress established fibrosis in different animal models of inflammatory and non-inflammatory SSc, could also lead to significantly enhanced wound healing in the TSK-mouse SSc model.

Taken together this data indicate for the first time that sGC stimulators and sGC activators, i.e. compounds according to formulae (27) and (3) could improve wound healing in an SSc. These data also suggest that despite the antifibrotic mode of action, these compounds are able to heal DUs in SSc patients.

### Disclosure of the invention

Fibrotic disorders addressed by therapeutic agents of the invention which in particular and with substantial advantage can be treated by the above mentioned sGC stimulators or sGC activators alone or in combination with PDE5 inhibitors comprise Systemic Sclerosis (SSc), Systemic Sclerosis (SSc) concomitant fibrosis and fibrotic diseases.

Fibrotic disorders addressed by therapeutic agents of the invention which in particular and with substantial advantage can be treated by the above mentioned sGC stimulators or sGC activators alone or in combination with PDE5 inhibitors comprise Systemic Sclerosis (SSc) concomitant vasculopathies, to Raynaud's phenomena (RD) and the formation and healing of digital ulcers (DU)

Systemic Sclerosis (SSc) refers to diffuse Systemic Sclerosis (dSSc), limited Systemic Sclerosis (lSSc), overlap type of Systemic Sclerosis, undifferentiated type of Systemic Sclerosis, Systemic Sclerosis sine scleroderma, skin fibrosis, scleroderma, nephrogenic fibrosing dermopathy (NFD), nephrogenic systemic fibrosis (NSF), keloid formation.

SSc concomitant fibrosis refers to fibrosis of internal organs, comprising the gut, the lung, the kidney and the blood vessels.

Fibrotic diseases comprise a condition in which collagen excess - independent of the etiology i.e. autoimmune disorders, chronic graft versus host disease, radiation therapy, intoxications, diabetes, surgery - lead to fibrosis of the skin, gut, liver, lung, heart, bladder, prostate, blood vessels or any other localized or generalized fibrotic condition in tissues.

In the sense of the present invention, the term fibrotic diseases comprises in particular the following terms: hepatic fibrosis, hepatic cirrhosis, pulmonary fibrosis, endomyocardial fibrosis, nephropathy, glomerulonephritis, interstitial renal fibrosis, fibrotic lesions as a consequence of diabetes, bone marrow fibrosis and similar fibrotic diseases, scleroderma, morphea, keloids, hypertrophic scars (including after surgery), naevi, diabetic retinopathy, proliferative vitreoretinopathy and connective tissue diseases (e.g. sarcoidosis

SSc concomitant vasculopathies comprise vascular occlusive diseases vasculitis, micro and macroangiopathies, Raynaud's Phenomena, digital ischemic lesions, digital ulcer, digital necrotic lesions, gangrene anddigital loss.

In the sense of the present invention, sGC stimulators are nitric oxide (NO) independent and heam-dependent modulators of the soluble guanylate cyclase.

In the sense of the present invention, sGC activators are nitric oxide (NO) and heam- independent modulators of the soluble guanylate cyclase.

The present disclosure describes the use of compounds according to compounds disclosed in WO03/097063, WO03/09545, WO04/009589, WO03/004503, WO02/070462, WO2007/045366, WO2007/045369, WO2007/045370, WO2007/045433, WO2007/045367, WO2007/124854, WO2007/128454, WO2008/031513, WO2008/061657, WO2008/119457, WO2008/119458, WO2009/127338, WO2010/079120, WO2010/102717, WO2011/051165, WO2011/147809, WO2011/141409, WO2014/012935, WO2012/059549, WO2012/004259, WO2012/004258, WO2012/059548, WO2012/028647, WO2012/152630, WO 2012/076466, WO2014/068099, WO2014/068104, WO2012/143510, WO2012/139888, WO2012/152629, WO2013/004785, WO2013/104598, WO2013/104597, WO2013/030288, WO2013/104703, WO2013/131923, WO2013/174736, WO2014/012934, WO2014/068095, WO2014/195333, WO2014/128109, WO2014/131760, WO2014/131741, WO2015/018808, WO2015/004105, WO2015/018814, WO98/16223, WO98/16507, WO98/23619, WO00/06569, WO01/19776, WO01/19780, WO01/19778, WO02/042299, WO02/092596, WO02/042300, WO02/042301, WO02/036120, WO02/042302, WO02/070459, WO02/070460, WO02/070461, WO02/070510, WO2012/165399, WO2014/084312, WO2011115804, WO2012003405, WO2012064559, WO2014/047111, WO2014/047325, WO2011/149921, WO2010/065275, WO2011/119518 for the manufacture of a medicament for prevention and healing of Digital Ulcers which are concomitant to fibrotic diseases, such as systemic sclerosis and scleroderma. An aspect of the present disclosure is the use of compounds according to formulae (1)-(28) for the manufacture of a medicament for prevention and healing of Digital Ulcers which are concomitant to fibrotic diseases, such as systemic sclerosis and scleroderma., as shown below:
2-[1-(2-Fluorbenzyl)-1H-pyrazolo[3,4-b]pyridine-3-yl]-5-(4-morpholinyl)-4,6-pyrimidinediamine (1), disclosed as example 16 in WO 00/06569,
- 2-[1-(2-Fluorobenzyl)-1H-pyrazolo[3,4-b]pyridine-3-yl]-5-(4-pyridinyl)-4-pyrimidine amine (2), disclosed as example 1 in WO 02/42301,
- Methyl-4,6-diamino-2-[1-(2-fluorobenzyl)-1H-pyrazolo[3,4-b]pyridine-3-yl]-5-pyrimidinyl-(methyl)carbamate (3), disclosed as example 8 in WO 03/095451,
- Methyl-4,6-diamino-2-[1-(2-fluorobenzyl)-1H-pyrazolo[3,4-b]pyridine-3-yl]-5-pyrimidinyl-carbamate (4), disclosed as example 5 in WO 03/095451
- 4-({(4-carboxybutyl)[2-(2-{[4-(2-phenylethyl)benzyl]oxy }phenyl)ethyl]amino}methyl) carboxylic acid (5), disclosed as example 8a in WO 01/019780,
- Methyl-{4,6-diamino-2-[5-fluoro-1-(2-fluorbenzyl)-1H-pyrazolo[3,4-b]pyridine-3-yl]pyrimidine-5-yl}carbamate (6), Methyl- {4,6-diamino-2-[5-fluoro-1-(2-fluorbenzyl)-1H-pyrazolo[3,4-b]pyridine-3-yl]pyrimidine-5-yl}methylcarbamate (7), Methyl-{4,6-diamino-2-[5-fluoro-1-(2-fluorbenzyl)-1H-pyrazolo[3,4-b]pyridine-3-yl]pyrimidine-5-yl}(2,2,2-trifluorethyl)carbamate (8), disclosed in WO 2011/147809,
- 5-Chloro-2-(5-chlorothiophene-2-sulfonylamino-N-(4-(morpholine-4-sulfonyl)-phenyl)-benzamid as sodium salt (9), disclosed in WO00/02851,
- 2-(4-Chloro-phenylsulfonylamino)-4,5-dimethoxy-N-(4-(thiomorpholine-4-sulfonyl)-phenyl)-benzamide (10), disclosed in WO00/02851,
- 1-{6-[5-Chloro-2-({4-trans-4-}trifluoromethyl)cyclohexyl]benzyl}oxy)phenyl]pyridine-2-yl}-5-(trifluoromethyl)-1H-pyrazol-4-carboxylic acid (11), disclosed in WO 2009/032249,
- 1- [6-(2-(2-Methyl-4-(4-trifluoromethoxyphenyl)benzyloxy)-phenyl)pyridine-2-yl] -5-trifluoromethyl-pyrazol-4-carboxylic acid (12), disclosed in WO 2009/071504,
- 1[6-(3,4-dichlorophenyl)-2-pyridinyl-5-(trifluoromethyl)-1H-pyrazole-4-caboxylic acid (13), disclosed in WO 2009/068652,
- 1-({2-[3-Chlor-5-(trifluoromethyl)phenyl]-5-methyl-1,3-thiazole-4-yl}methyl)-1H-pyrazole-4-carboxylic acid (14), 4-({2-[3-(Trifluoromethyl)phenyl]-1,3-thiazole-4-yl}methyl)benzoic acid (15) and 1-({2-[2-Fluoro-3-(trifluoromethyl)phenyl]-5-methyl-1,3-thiazole-4-yl}methyl)-1H-pyrazole-4-carboxylic acid (16) disclosed in WO 2009/123316,
- 4-Amino-2-[5-chloro-3(3,3,3-trifluororpropyl)-1H-indazol-1yl]-5,5-dimethyl-5,7-dihydro-6H-pyrrolo[2,3-d]pyrimidine-6-one (17), 4-Amino-2[5-chloro-3-(2,3,6-trifluorbenzyle)-1H-indazol-1yl]-5,5-dimethyl-5,7-dihydro-6H-pyrrolo[2,3-d]pyrimidine-6-one (18), 4-Amino-5,5-dimethyl-2-[3-(2,3,6-trifluorbenzyle)1H-thieno[3,4-c]pyrazol-1-yl]-5,7-dihydro-6H-pyrrolo[2,3-d]pyrimidine-6-one (19), 4-Amino-5,5-dimethyl-2-[3-(2,3,6-trifluorbenzyle)-1H-thieno[2,3-d]pyrazole-1-yl]-5,5-dimethyl-5,7-dihydro-6H-pyrrolo[2,3-d]pyrimidine-6-one (20), 4-Amino-5,5-dimethyl-2-[7-(2,3,6-trifluorobenzyle)imidazo[1,5-b]pyridazine-5-yl]-5,7-dihydro-6H-pyrrolo[2,3-d]pyrimidine-6-one (21), 4-Amino-2-[6-chloro-3-(2,3,6-trifluorobenzyle)imidazo[1,5-a]pyridine-1-yl]]-5,5-dimethyl-5,7-dihydro-6H-pyrrolo[2,3-d]pyrimidine-6-one (22), 4-Amino-2-[6-fluoro-3-(2,3,6-trifluorobenzyle)imidazo[1,5-a]pyridine-1-yl]]-5,5-dimethyl-5,7-dihydro-6H-pyrrolo[2,3-d]pyrimidine-6-one (23), 4-Amino-2-[6-fluoro-3-(2,3,6-trifluorobenzyl)6-fluoroimidazo[1,5-a]pyridine-1-yl]-5,5-dimethyl-5,7-dihydro-6H-pyrrolo[2,3-d]pyrimidine-6-one (24), 4-Amino-5,5-dimethyl-2-[3-(2,4,6-trifluorobenzyle)imidazo[1,5-a]pyridine-1-yl]]-5,7-dihydro-6H-pyrrolo[2,3-d]pyrimidine-6-one (25), 4-Amino-2-[3-(2-cyclopentylethyl)imidazo[1,5-a]pyridine-1-yl]-5,5-dimethyl-5,7-dihydro-6H-pyrrolo[2,3-d]pyrimidine-6-one (26), disclosed in WO 2010/065275,
- 3-(4-Amino-5-cyclopropylpyrimidine-2-yl)-1-(2-fluorobenzyl)-1*H*-pyrazolo[3,4-*b*]pyridine (27) known as BAY 41-2272 disclosed as example 1 in WO 00/06568,
- 2-{5-Fluor-1-[(3-fluorpyridine-2-yl)methyl]-1H-pyrazolo[3,4-b]pyridine-3-yl}-5-methyl-5-(trifluormethyl)-4-[(3,3,3-trifluorpropyl)amino]-5,7-dihydro-6H-pyrrolo[2,3-d]pyrimidine-6-on (28), disclosed as example 1 in WO 2014/131760.

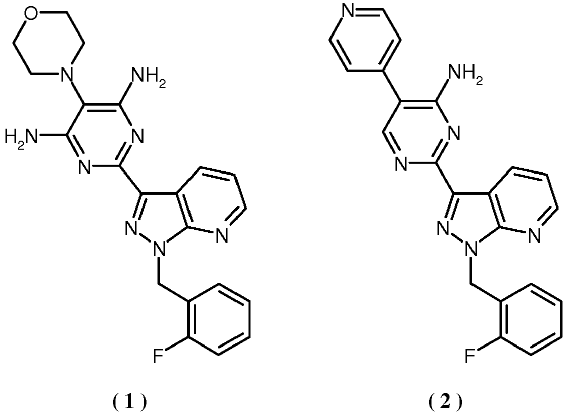

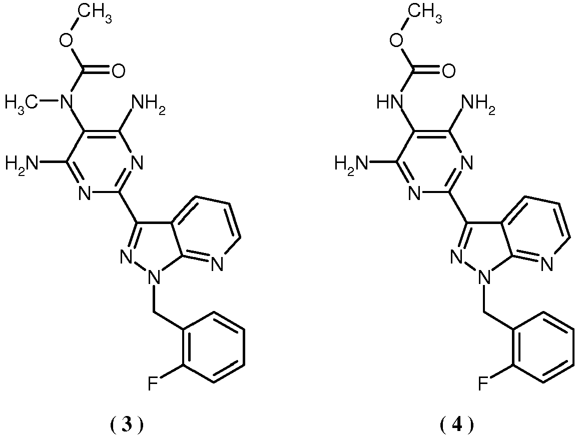

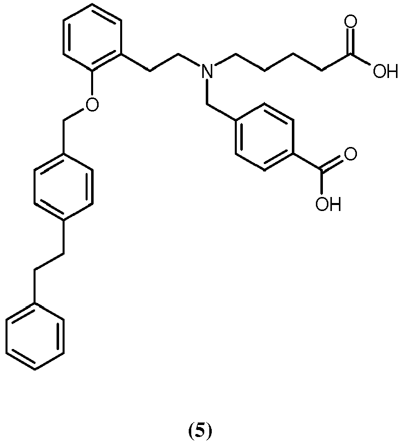

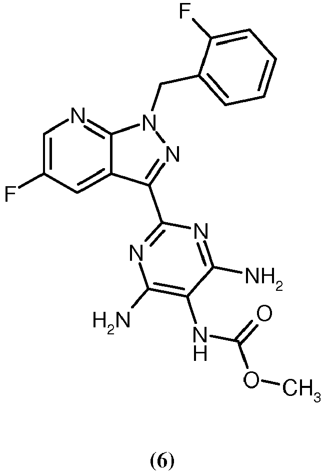

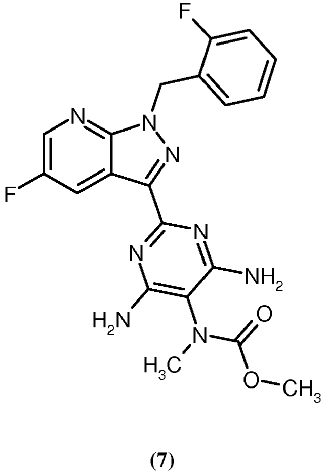

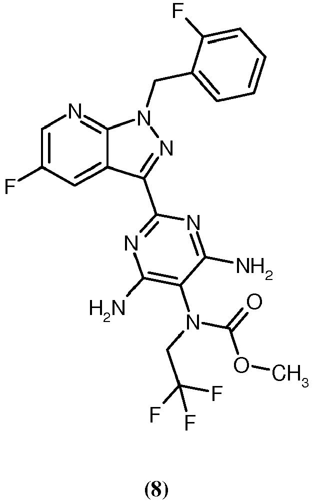

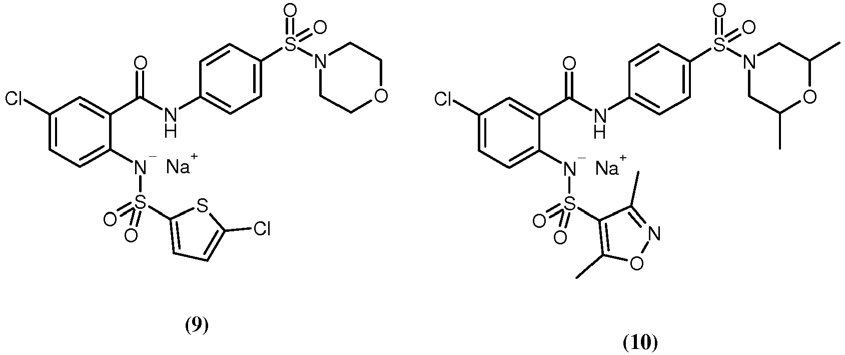

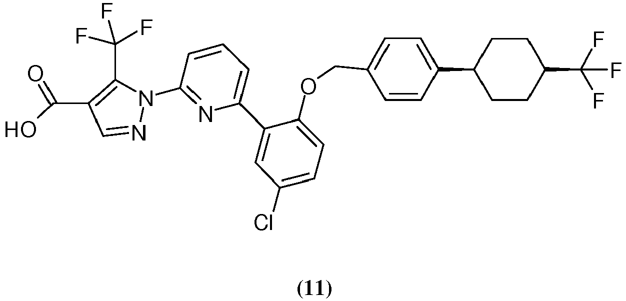

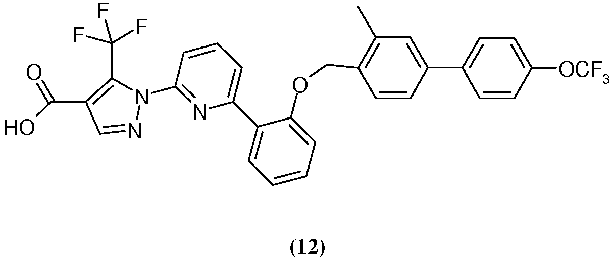

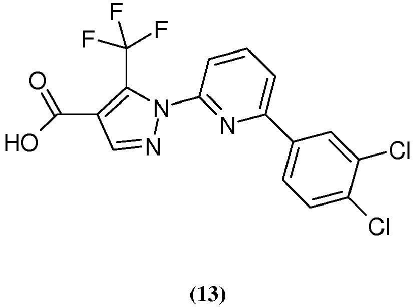

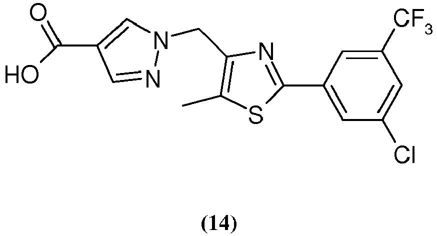

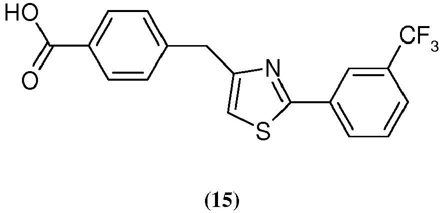

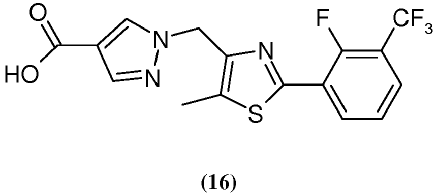

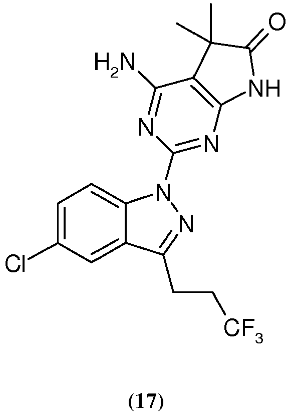

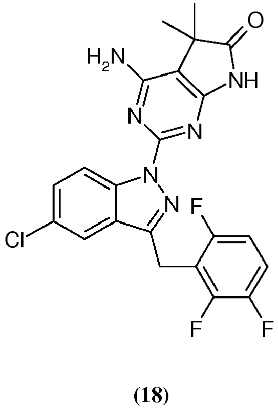

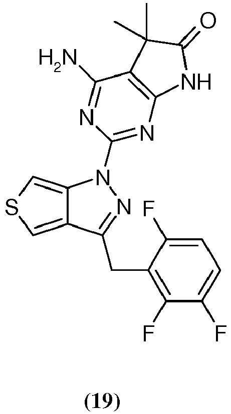

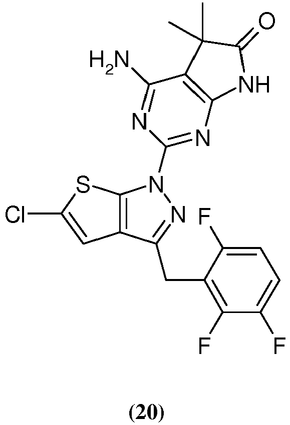

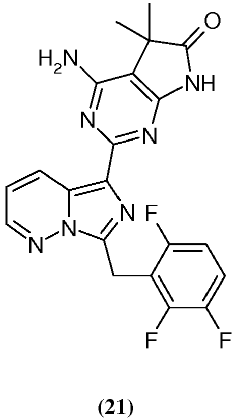

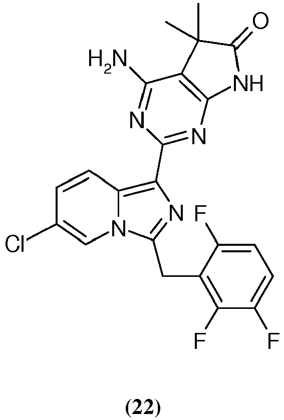

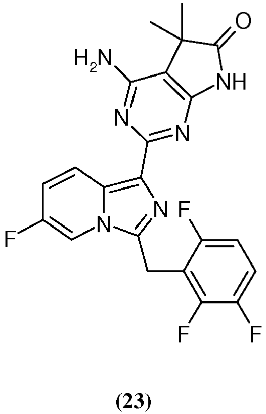

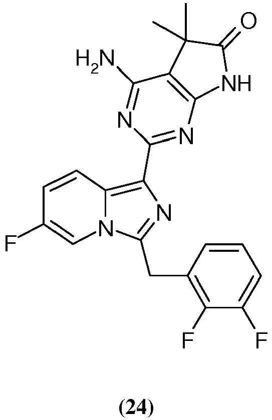

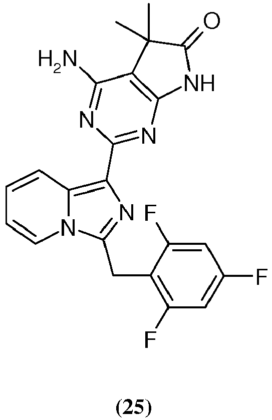

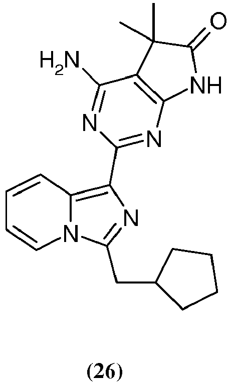

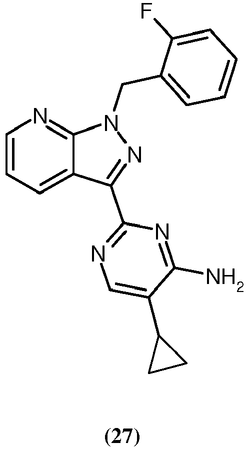

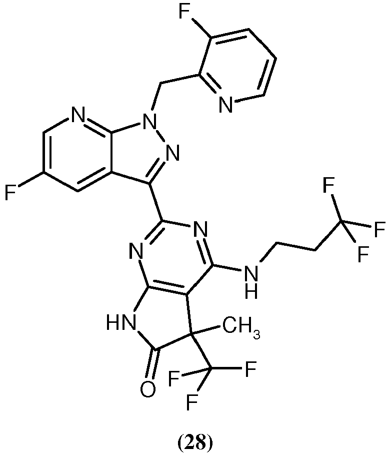

Compounds according to formulae (1), (2), (3), (4), (6)-(8) and (17)-(27) are known as sGC stimulators. According to the invention, the compounds for use in the acceleration of wound healing in patients with Digital Ulcers concomitant to fibrotic diseases, such as systemic sclerosis and scleroderma are compounds according to formulae (3), (6) and (27).

Especially preferred is the use of the compound according to formula (3).

Compounds according to formulae (5) und (9)-(16) are known as sGC activators. A further embodiment of the invention is a combination of stimulators and/or activators of the soluble guanylate cyclase (3), (6) or (27) withPDE5 inhibitors for use for the healing of Digital Ulcers which are concomitant to fibrotic diseases, such as systemic sclerosis and scleroderma.

The use of the following PDE 5 inhibitors is preferred for the combination with sGC stimulators and/or activators:
*Tadalafil* ((6R,12aR) -2,3,6,7,12,12a - Hexahydro - 2 - methyl - 6 - (3,4-methylene -dioxyphenyl) pyrazino(1',2':1,6) pyrido(3,4-b)indole-1,4-dione), *Vardenafil* (2-(2-Ethoxy-5-(4-ethylpiperazin-1-yl-1-sulfonyl)phenyl)-5-methyl-7-propyl-3H-imidazo (5,1-f) (1,2,4)triazin-4-one), *Sildenafil (3-[2-*ethoxy-5-(4-methylpiperazin-1-yl)sulfonyl-phenyl]-7- methy 1- 9- propy 1-2,4,7,8- tetrazabicyclo [4.3.0]nona -3,8,10-trien-5-one), *Udenafil* 5-[2-propyloxy-5-(1-methyl-2-pyrrolidinylethylamidosulfonyl)phenyl]-methyl-3-propyl-1,6-dihydro-7H-pyrazolo(4,3-d)pyrimidine-7-one, *Dasantafil* 7-(3-Bromo-4-methoxybenzyl)-1-ethyl-8-[[(1,2)-2-hydroxycyclopentyl]amino]-3-(2-hydroxyethyl)-3,7-dihydro-1-purine-2,6-dione, *Avanafil* 4-{[(3-chloro-4-methoxyphenyl)methyl] amino}-2-[(2S)-2-(hydroxymethyl)pyrrolidin-1-yl] -N-(pyrimidin-2-ylmethyl)pyrimidine-5-carboxamide, Mirodenafil, Lodenafil, UK 369.003, UK 371.800, *SLx* 2101 of Surface Logix, *LAS 34179*Triazolo[1,2-]xanthine,6-methyl-4-propyl-2-[2-propoxy-5-(4-methylpiperazino)sulfonyl]phenyl or salts, hydrates or hydrates of the salts.

Especially preferred is the use of combinations of compounds according to formulae (3), (6), (27), with vardenafil and/or sildenafil for the healing of Digital Ulcers which are concomitant to fibrotic diseases, such as systemic sclerosis and scleroderma.

Especially preferred is the use of compounds according to formulae (3), and/or (6) for the healing of Digital Ulcers which are concomitant to fibrotic diseases, such as systemic sclerosis and scleroderma.

Especially preferred is the use of at least one compound according to formulae (3), and/or (6), in combination with vardenafil or sildenafil for the healing of Digital Ulcers which are concomitant to fibrotic diseases, such as systemic sclerosis and scleroderma.

The sGC stimulator such as compound according to formula (3) dose-dependently and significantly accelerated wound healing in the tsk-1 skin fibrosis model in mice. The tsk-1 mouse model is characterized by substantial skin fibrosis reflecting a non-inflammatory driven, stable SSc phenotype. These data imply that the sGC stimulators such as compound according to formula (3) could become an efficacious treatment option for SSc-related vasculopathies, especially for prevention and healing of Digital Ulcer.

The compounds according to the invention can be used alone or in combination with other active substances if necessary. The present invention further relates to medicinal products containing at least one of the compounds according to the invention and one or more further active substances, in particular for the treatment and/or prophylaxis of the aforementioned diseases. As suitable combination active substances, we may mention for example and preferably:
- organic nitrates and NO-donors, for example sodium nitroprusside, nitroglycerin, isosorbide mononitrate, isosorbide dinitrate, molsidomine or SIN-1, and inhalational NO;
- other vasoactive drugs, for examples prostanoids, such as iloprost, beraprost, cicaprost, epoprostenol, treprostinil;
- other vasoactive drugs, for example Rho-kinase inhibitors such as fasudil;
- other vasoactive drugs, for example endothelin receptor antagonists such as bosentan, darusentan, ambrisentan or sitaxsentan, macitentan;
- active substances for lowering blood pressure, for example and preferably from the group of calcium antagonists, such as nifedipine, amlodipine, verapamil or diltiazem;
- active substances for lowering blood pressure, for example and preferably from the group of angiotensin All antagonists, ACE inhibitors, renin inhibitors, alpha-blockers, beta-blockers, mineralocorticoid receptor antagonists and diuretics; and/or
- antithrombotic agents, for example and preferably from the group of platelet aggregation inhibitors, anticoagulants, thrombin inhibitors or profibrinolytic substances;
- active substances that alter fat metabolism, for example and preferably from the group of thyroid receptor agonists, cholesterol synthesis inhibitors such as for example and preferably HMG-CoA-reductase or squalene synthesis inhibitors, ACAT inhibitors, CETP inhibitors, MTP inhibitors, PPAR-alpha, PPAR-gamma and/or PPAR-delta agonists, cholesterol absorption inhibitors, lipase inhibitors, polymeric bile acid adsorbers, bile acid reabsorption inhibitors and lipoprotein(a) antagonists;
- active substances that are used in fibrotic disorders, for examples and preferable from the group of proteinkinase inhibitors such as sorafenib, regorafenib, imatinib, dasatinib, nilotinib nintedanib, bortezomib and/or pirfenidone;
- active substances that alter inflammatory responses and/or supress immune responses, for example such as, cyclophosphamide, methotrexate, rapamycin, azathioproin, tocilizumab, infliximab, rituximab, adalimumab, belimumab, abatacept, SAR100842, thalidomide derivates;
- active substances working on different pathways, for example pirfenidone, SAR100842, thalidomide derivatives, integrin inhibitors.

Another preferred embodiment of the invention are compounds and/or combinations indicated above for use in the healing of Digital Ulcers which are concomitant to fibrotic diseases, such as systemic sclerosis and/or scleroderma.

Another preferred embodiment of the invention is the pharmaceutical formulation comprising at least one compound or one combination as indicated above for the use in the healing of Digital Ulcers which are concomitant to fibrotic diseases, such as systemic sclerosis and/or scleroderma.

Another preferred embodiment of the invention is a kit comprising at least one sGC stimulator and/or activator as indicated above or a combination as indicated above for the use in the healing of Digital Ulcers which are concomitant to fibrotic diseases, such as systemic sclerosis and/or scleroderma.

A pharmaceutical composition of the invention is formulated to be compatible with its intended route of administration. Examples of routes of administration include parenteral e.g., intravenous, intradermal, subcutaneous' oral (e.g.' inhalation)' transdermal (topical) transmucosal and rectal administration. Pharmaceutical compositions suitable for injectable use include sterile aqueous solutions (where water soluble) or dispersions and sterile powders for the extemporaneous preparation of sterile injectable solutions or dispersions. The carrier can be a solvent or dispersion medium containing, for example, water, ethanol, a pharmaceutically acceptable polyol like glycerol, propylene glycol, liquid polyetheylene glycol, and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents for example, parabens, chlorobutanol, phenol, ascorbic acid, thimerosal, and the like. In many cases, it will be preferable to include isotonic agents, for example, sugars, polyalcohols such as maitol sorbitol sodium chloride in the composition.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed.

Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or con1 starch; a lubricant such as magnesium stearate or sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from a pressurized container or dispenser which contains a suitable propellant, e.g.' a gas such as carbon dioxide, or a nebulizer.

Systemic administration can also be by transmucosal or transderrnal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Bio degradable, biocompatible polymers can be used, such as ethylene vinyl acetate, polyanhydrides, polyglycolic acid, collagen, polyorthoesters, and polylactic acid.

### References:

Beyer C, Schett G, Distler O, Distler JH (2010): Animal models of systemic sclerosis: prospects and limitations. Arthritis Rheum. 62(10):2831-44
Evgenov OV, Pacher P, Schmidt PM et al. (2006): NO-independent stimulators and activators of soluble guanylate cyclase: discovery and therapeutic potential. Nat. Rev. Drug. Discov. 5(9):755-68
Ferrini MG, Kovanecz I, Nolazco G (2006): Effects of long-term vardenafil treatment on the development of fibrotic plaques in a rat model of Peyronie's disease. B. J. U. 97:625-633.
Harris ED, et al. (2005): Kelley's Textbook of Rhematology 7th edition. Elsevier Saunders, Philadelphia PA
Kaplan SA, Gonzalez RR (2007): Phosphodiesterase type 5 inhibitors for the treatment of male lower urinary tract symptoms. Rev. Urol. 9(2):73-77
Khanna D and Denton CP (2010) Evidence-based management of rapidly progressing systemic sclerosis. Best. Pract. Res. Clin. Rheumatol. 24:387-400
Knorr A, Hirth-Dietrich C, Alonso-Alija C. et al. (2008): Nitric oxide-independent activation of soluble guanylate cyclase by BAY 60-2770 in experimental liver fibrosis. Arzneimittelforschung 58:71-80.
MVary K K. T. McVary, W. Monnig, J. L. Camps, Jr., J. M. Young, L. J. Tseng and G. van den Ende (2007): Sildenafil citrate improves erectile function and ur inary symptoms in men with erectile dysfunction and lower urinary tract symptoms associated with benign prostatic hyperplasia: a randomized, double-blind trial. J. Urol. 177: 1071-1077.
McVary KT, Roehrborn CG, Kaminetsky JC, Auerbach SM, Wachs B, Young JM, Esler A, Sides GD, Denes BS. (2007): Tadalafil relieves lower urinary tract symptoms secondary to benign prostatic hyperplasia. J Urol. 177:1401-1407.
Ong VH and Denton CP (2010): Innovative therapies for systemic sclerosis Curr. Opin. Rheumatol. 22:264-272.
Porst H, Sandner P, Ulbrich E. (2008): Vardenafil in the treatment of lower urinary tract symptoms secondary to benign prostatic hyperplasia. Curr. Urol. Rep. 9:295-301.
Sandner P, Neuser D, Bischoff E (2009): Erectile dysfunction and lower urinary tract. Handb. Exp. Pharmacol. 191:507-531.
Spiera R, Gordon J, Mersten J, Magro C, Mehta M, Wildmann H, Kloiber S, Kirou K, Lyman S, Crow M (2011): Imatinib mesylate (Gleevec) in the treatment of diffuse cutaneous systemic sclerosis: results of a lyear, phse IIa, single-arm open-label clinical trial. Ann. Rheum. Dis. Epub Mar 11, 2011

### Figures:

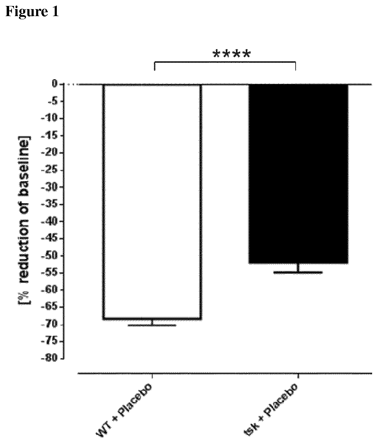

Figure 1: Reduction of wound size in WT mice (left) and tsk-1 mice (right) treated with placebo after three days. Data are mean - SEM, n=46 (WT + Placebo) and n=44 (tsk-1 + Placebo), */**/***/****= significant with p<0.05/0.01/0.001/0.0001

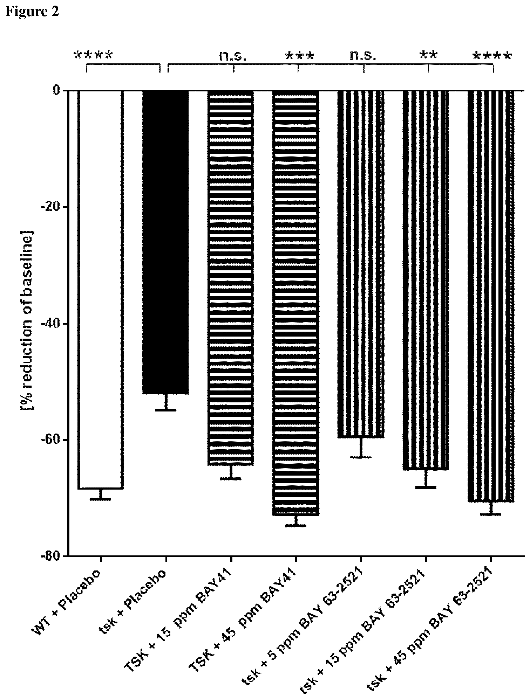

Figure 2: Reduction of wound size in tsk-1 mice treated with either placebo or compound according to formula (27), (BAY 41-2272) or compound according to formula (3), (BAY 63-2521) after three days. Data are mean - SEM, n=44-46 (Placebo groups), n= 16 (compound according to formula (27) BAY 41-2272 groups) and n=30-32 (compound according to formula (3) BAY 63-2521 groups), */**/***/****= significant with p<0.05/0.01/0.001/0.0001; ns = non significant; BAY41 corresponds to BAY41-2227.

### Experimental Part

### Example A

### Wound Healing in Tsk-1 mice versus WT-mice

The tight-skin (Tsk-1) mouse model of SSc was used to evaluate the effects of compound according to formula (27) and (3) (BAY 41-2272 and BAY 63-2521) on wound healing in mice with substantial skin fibrosis. Due to an autosomal dominant mutation namely a tandem duplication of the fibrillin-1 gene, the phenotype of tsk-1 mice is characterized by an increased hypodermal thickness (Beyer et al. 2010). Genotyping of Tsk-1 mice was performed by PCR with the following primers: mutated fibrillin-1/ tsk-1 forward primer: 5' - GTTGGCAACTATACCTGCAT - 3', reverse primer: 5' - CCTTTCCTGGTAACATAGGA - 3'.

The effects of placebo (= vehicle for the test compounds = 0.5 tylose solution) was studied in either WT mice or in Tsk-1 mice. Tsk-1 mice were anaesthetized and carefully shaved 3 days before setting the wounds for exact quantification of the wound size. In order to avoid influences on wound healing by daily handling of the animals, the usual bi-daily gavage treatment was replaced by drug administration in the food. WT mice and Tsk-1 mice received normal mice chow (placebo) which started on the day of shaving. Three days after shaving, mice were carefully anesthetized and round wounds were punched with 4 mm in diameter. 3 days after punching, mice were euthanized and the wound size was assessed. Statistical analysis of data was done by one-way ANOVA followed by Tuckey's multiple comparison post-hoc analysis

In tsk-1 mice, wound sizes were reduced by 52% ± 2 % after placebo treatment (Figure 1). In contrast, placebo treated WT mice showed a reduction in wound size of 68% ± 2% after 3 days (Figure 1). Therefore, wound healing in tsk-1 mice was partly impaired compared to WT mice and wound closure was significantly attenuated in placebo-treated tsk-1 mice.

### Wound Healing in Tsk-1 mice treated with the compound according to formula (27) and (3), (BAY 41-2272 and BAY 63-2521)

The effects of either placebo (= vehicle for the compounds = 0.5% tylose solution), or the compound according to formula (27) or (3), (BAY 41-2272 or BAY 63-2521) were studied in Tsk-1 mice. Tsk-1 mice were anaesthetized and carefully shaved 3 days before setting the wounds for exact quantification of the wound size. In order to avoid influences on wound healing by daily handling of the animals, the bi-daily gavage treatment was replaced by drug administration in the food. Mice received either normal mice chow (placebo) or mice chow, containing 15 and 45 ppm of compound according to formula (27), (BAY 41-2272) or containing 5ppm, 15 ppm and 45 ppm of compound according to formula (3), (BAY 63-2521), respectively. These dosages - as confirmed by an orientating DMPK-study - resulted in similar exposures as 1 and 3 mg/kg of compound according to formula (27), (BAY 41-2272) BID and 0.3, 1 and 3 mg/kg of compound according to formula (3), (BAY 63-2521) BID, respectively. Treatment groups consist of at least 8 tsk-1 mice per group. Treatment started on the day of shaving to achieve steady state exposure. Three days after shaving, mice were carefully anesthetized and round wounds were punched with 4 mm in diameter. 3 days after punching, mice were euthanized and the wound size was assessed. Statistical analysis of data was done by one-way ANOVA followed by Tuckey's multiple comparison post-hoc analysis

In the tsk-1 mice wound sizes were dose-dependently and significantly reduced by 64 ± 2% and by 73 ± 2% after treatment with 15 and 45 ppm of compound according to formula (27), (BAY 41-2272), respectively (Figure 2). In addition, in the tsk-1 mice wound sizes were dose-dependently and significantly reduced by 59% ± 4%, 65 ± 3% and 70% ± 2% after treatment with 5, 15 and 45 ppm of compound according to formula (3), (BAY 63-2521), respectively (Figure 2). In addition, treatment with 45 ppm of compound according to formula (27), (BAY 41-2272) and 45 ppm of compound according to formula (3), (BAY 63-2521, Riociguat) normalized wound healing to a similar extent as observed in placebo-treated WT mice (68% ± 2%) (Figure 1, Figure 2). Therefore, compound according to formula (27) and (3), (BAY 41-2272 and BAY 63-2521) accelerated wound healing in compared to placebo treatment in the TSK-mice and lead to a normalization of wound closure as found in healthy control mice.

### In summary, these data indicated that:

a) Wound healing in to tsk-1 mice was significantly attenuated compared to WT mice.
b) Wound healing in TSK-1 mice was significantly and dose-dependently improved by treatment with compound according to formula (27), (BAY 41-2272) and/or compound according to formula (3) BAY 63-2521.
   - Maximum efficacy lead to a wound closure which was similar to healthy WT mice.
      Since Tsk-1 mice are an animal model with extensive skin fibrosis reflecting the conditions in SSc this data indicate that the aforementioned compounds could not only reduce fibrosis but also accelerate wound healing in SSc, implying that these compounds are useful for the treatment of DU in SSc patients.

## Claims

1. At least one compound selected from the group consisting of Methyl-4,6-diamino-2-[1-(2-fluorobenzyl)-1H-pyrazolo [3,4-b]pyridine-3 -yl] -5 -pyrimidinyl(methyl)carbamate (3), Methyl- {4,6-diamino-2-[5-fluoro-1-(2-fluorobenzyl)-1H-pyrazolo[3,4-b]pyridine-3-yl]pyrimidine-5-yl}carbamate (6), and 3-(4-Amino-5-cyclopropylpyrimidine-2-yl)-1-(2-fluorobenzyl)-1*H*-pyrazolo[3,4-*b*]pyridine (27) for use in the acceleration of wound healing in patients with Digital Ulcers concomitant to fibrotic diseases, such as systemic sclerosis and scleroderma.

2. Methyl-4,6-diamino-2-[1-(2-fluorobenzyl)-1H-pyrazolo[3,4-b]pyridine-3-yl]-5-pyrimidinyl(methyl)carbamate (3) for use in the acceleration of wound healing in patients with Digital Ulcers concomitant to fibrotic diseases, such as systemic sclerosis and scleroderma.

## Patentansprüche

1. Wenigstens eine Verbindung, ausgewählt aus der Gruppe bestehend aus Methyl-4,6-diamino-2-[1-(2-fluorbenzyl)-1H-pyrazolo[3,4-b]pyridin-3-yl]-5-pyrimidinyl(methyl)carbamat (3), Methyl-{4,6-di-amino-2-[5-fluor-1-(2-fluorbenzyl)-1H-pyrazolo-[3,4-b]pyridin-3-yl]pyrimidin-5-yl}carbamat (6) und 3-(4-Amino-5-cyclopropylpyrimidin-2-yl)-1-(2-fluorbenzyl)-1H-pyrazolo[3,4-*b*]pyridin (27), zur Verwendung bei der Beschleunigung der Wundheilung bei Patienten mit digitalen Ulzera (Digital Ulcers) als Begleiterscheinung von Fibrosen wie systemischer Sklerose und Sklerodermie.

2. Methyl-4,6-diamino-2-[1-(2-fluorbenzyl)-1H-pyrazolo[3,4-b]pyridin-3-yl]-5-pyrimidinyl-(methyl)carbamat (3) zur Verwendung bei der Beschleunigung der Wundheilung bei Patienten mit digitalen Ulzera (Digital Ulcers) als Begleiterscheinung von Fibrosen wie systemischer Sklerose und Sklerodermie.

## Revendications

1. Composé (s) choisi (s) dans le groupe constitué par le 4,6-diamino-2-[1-(2-fluorobenzyl)-1H-pyrazolo[3,4-*b*]pyridin-3-yl]-5-pyrimidinyl(méthyl)carbamate de méthyle (3), le {4,6-diamino-2-[5-fluoro-1-(2-fluorobenzyl)-1*H-*pyrazolo[3,4-b]pyridin-3-yl]-pyrimidin-5-yl}carbamate de méthyle (6) et la 3-(4-amino-5-cyclopropylpyrimidin-2-yl)-1-(2-fluorobenzyl)-1*H-*pyrazolo[3,4-*b*]pyridine (27) pour une utilisation dans l'accélération de la cicatrisation des plaies chez des patients possédant des ulcères digitaux concomitants à des maladies fibrotiques, telle que la sclérose systémique et la sclérodermie.

2. 4,6-diamino-2-[1-(2-fluorobenzyl)-1*H*-pyrazolo[3,4-*b*]pyridin-3-yl]-5-pyrimidinyl(méthyl)carbamate de méthyle (3) pour une utilisation dans l'accélération de la cicatrisation des plaies chez des patients possédant des ulcères digitaux concomitants à des maladies fibrotiques, telle que la sclérose systémique et la sclérodermie.


### SEQUENCE LISTING

<110> Bayer Pharma AG
<120> The use of sGC stimulators, sGC activators, alone and combinations with PDE5 inhibitors for the treatment of Digital Ulcers (DU) concomitant to systemic sclerosis (SSc)
<130> BHC141003
<160> 2
<170> PatentIn version 3.5
<210> 1
   <211> 20
   <212> DNA
   <213> Artificial Sequence
<220>
   <223> mutated fibrillin-1/ tsk-1 forward primer
<400> 1
   gttggcaact atacctgcat 20
<210> 2
   <211> 20
   <212> DNA
   <213> Artificial Sequence
<220>
   <223> reverse primer
<400> 2
   cctttcctgg taacatagga 20